# EUROPEAN PATENT SPECIFICATION

(11) **EP 1 513 519 B1**
(45) Date of publication and mention of the grant of the patent: **18.02.2009**
(21) Application number: 03737984.9
(22) Date of filing: 02.06.2003
(51) Int. Cl.: A61K 31/40, A61P 9/10

(54) **THE USE OF SUBSTITUTED CYANOPYRROLIDINES FOR TREATING HYPERLIPIDEMIA**
VERWENDUNG VON SUBSTITUIERTEN CYANOPYRROLIDINEN ZUR BEHANDLUNG VON HYPERLIPIDÄMIE
UTILISATION DE CYANOPYRROLIDINES SUBSTITUEES DANS LE TRAITEMENT D'HYPERLIPIDEMIE

(30) Priority: 03.06.2002 US 385220 P
(43) Date of publication of application: 16.03.2005
(73) Proprietor: NOVARTIS AG, 4056 Basel (CH); Novartis Pharma GmbH, 1230 Wien (AT)
(72) Inventor: HOLMES, David, Grenville, CH-4102 Binningen (CH); HUGHES, Thomas, Edward, Concord, MA 01742 (US)
(74) Representative: Hillebrand, Dirk
(86) International application number: PCT/EP2003/005762
(87) International publication number: WO 2003/101448

(56) References cited:
- EP-A- 0 187 052
- EP-A- 1 125 922
- WO-A-00/34241
- WO-A-03/038123
- WO-A-03/080070
- WO-A-03/099279
- US-A- 6 011 155
- US-A- 6 166 063

## Description

Hyperlipidemia is an important precipitating factor for the premature development of atherosclerosis and increased rate of cardiovascular and peripheral vascular diseases. Hyperlipidemia is a condition generally characterized by an abnormal increase in serum lipids in the bloodstream and is an important risk factor in developing atherosclerosis and heart disease. For a review of disorders of lipid metabolism, see, e.g., Wilson, et al., Ed., Disorders of Lipid Metabolism, Chapter 23, Textbook of Endocrinology, 9th Edition, W.B. Sanders Company, Philadelphia, PA (1998). Serum lipoproteins are the carriers for lipids in the circulation and include chylomicrons, very low-density lipoproteins (VLDL), intermediate density lipoproteins (IDL), low density lipoproteins (LDL) and high density lipoproteins (HDL) and lipoprotein a (Lp(a)). Hyperlipidemia is usually classified as primary or secondary hyperlipidemia. Primary hyperlipidemia is generally caused by genetic defects, while secondary hyperlipidemia is generally caused by other factors, such as various disease states, drugs and dietary factors. Alternatively, hyperlipidemia can result from both a combination of primary and secondary causes of hyperlipidemia. Elevated cholesterol levels are associated with a number of disease states, including coronary artery disease, angina pectoris, carotid artery disease, strokes, cerebral arteriosclerosis, and xanthoma.

There are several forms of circulating blood cholesterol which occur naturally in mammals. Some forms are considered "bad" cholesterol, while other forms are considered "good" cholesterol and are essential for good health. The good form of cholesterol has been established to be HDL. LDL is a "bad" cholesterol. Another form of LDL cholesterol, the primary bad form, is Lp(a) which is a modified form of LDL. Elevated levels of Lp(a) are believed to be detrimental and associated with a higher risk for coronary heart disease (CHD) (see Assman et al., Am. J. Card., Vol. 77, pp. 1179-1184 (1996); and Bostom et al., JAMA, Vol. 276, No. 7, pp. 544-548 (1996)). Lowering of Lp(a) levels with a combination of estrogen and progesterone is associated with a lower incidence of detrimental coronary events (see Shlipak et al., JAMA, Vol. 283, No. 14, pp. 1845-1852 (2000)).

Lowering LDL, the bad form of cholesterol, is now one of the primary objectives of physicians treating patients who have, or who have a high risk of developing, cardiovascular diseases, such as CHD, atherosclerosis, myocardial infarction, stroke, cerebral infarction, and even restenosis following balloon angioplasty. Many physicians are now utilizing cholesterol-lowering agents purely as a prophylactic treatment in healthy subjects whose cholesterol levels are normal, thereby guarding against development of cardiovascular diseases.

WO-A0034241 describes compounds of formula (A) 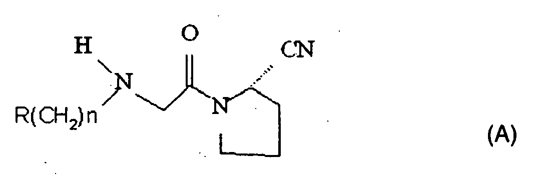 wherein R is substituted adamantyl; and n is 0 to 3; in free form or in acid addition salt form and their use as pharmaceuticals in inhibiting DPP-IV and in the treatment of conditions mediated by DPP-IV, such as non-insulin-dependent diabetes mellitus, arthritis, obesity, osteoporosis and further conditions of impaired glucose tolerance.

WO-A-03038123 describes the use of the novel association between the 483 A>G single nucleotide polymorphism of the TCF1 gene and the clinical response to glycemic control agents, such as DPPIV inhibitors, e.g. 1-[3Hydroxy-adamant-1-ylamino)-acetyl]-pyrrolidine-2 (S)-carbonitrile, in patients with disorders of glycemic control, especially diabetes and impaired glucose metabolism.

EP-A-0187052 describes adamantanamine derivatives and their use as anticonvulsants.

EP-A-1125922 describes pyrrolidine compounds having a serotonin 2 receptor antagonistic action along with a platelet aggregation suppressive action, a peripheral circulation improving action and a lacrimation promoting action and their use against thrombotic embolism, dry eye and the like.

WO-A-03099279 (an Article 54(3)-document) describes combination preparation comprising of a DPP IV inhibitor (such as S)-1- [(3-hydroxy-1-adamantyl) amino]acetyl-2- cyano-pyrrolidine) in combination with AT₁-receptor antagonist such as losartan, olmesartan orvalsartan; ACE inhibitor such as benazepril, enalapril, lisinopril or ramipril ; renin inhibitor such as aliskiren ; or beta blockersuch as metoprolol and the use of said combination for the treatment of (a) type 2 diabetes mellitus and related diseases, disorders or conditions; (b) insulin resistance and syndrome X, obesity (c) hypertension including hypertension in the elderly, familial dyslipidemic hypertension, and isolated systolic hypertension(ISH) ; increased collagen formation, fibrosis, and remodeling following hypertension; erectile dysfunction, impaired vascular compliance, stroke; all these diseases or conditions associated with or without hypertension, (d) congestive heart failure, left ventricular hypertrophy, survival post myocardial infarction(MI), coronary artery diseases, atherosclerosis, angina pectoris, thrombosis, (e) renal failure, especially chronic renal failure, glomerulosclerosis, nephropathy; (f) hypothyroidism; (g) endothelial dysfunction with or without hypertension, (h) hyperlipidemia, hyperlipoproteinemia, hypertryglyceridemia, andhypercholesterolemia, (i) macular degeneration, cataract, glaucoma,(j) skin and connective tissue disorders, and (k) restenosis after percutaneous transluminal angioplasty, and restenosis after coronary artery bypass surgery; peripheral vascular disease.

WO-A-03080070 (an Article 54(3)-document) describes a combination comprising a HMG CoA reductase inhibitor selected from the group consisting of atorvastatin, cerivastatin, fluvastatin, lovastatin, pitavastatin, pravastatin, rosuvastatin, and simvastatin, and an insulin secretion enhancer such as pyrrolidine, 1-[(3-hydroxy-1-adamantyl) amino] acetyl-2-cyano-, (S) and the use of said combination in the prevention of, delay of progression of, treatment of a disease or condition selected from the group consisting of hyperlipidaemia, dyslipidemia, atherosclerosis, insulin resistance and syndrome X, diabetes mellitus type 2, obesity, nephropathy, renal failure, hypothyroidism, survival post myocardial infarction(MI), coronary heart diseases, hypertension in the elderly, familial dyslipidemic hypertension, remodeling following hypertension, non alcoholic fatty liver disorders, polycystic ovary syndrome (PCOS).

The most commonly used cholesterol-lowering agents are the statins, which are compounds which inhibit the enzyme 3-hydroxy-3-methylglutarylcoenzyme A (HMG-CoA) reductase, the enzyme responsible for catalyzing the conversion of HMG-CoA to mevalonate, which is an early and rate-limiting step in the cholesterol biosynthetic pathway.

Due to these debilitating effects of hyperlipidemia, there is a need for new therapeutic methods and compositions for modulating, treating or preventing hyperlipidemia and conditions associated therewith.

Toward these ends and others, the present invention relates to the use as claimed in claim 1.

Compound of formula IC: 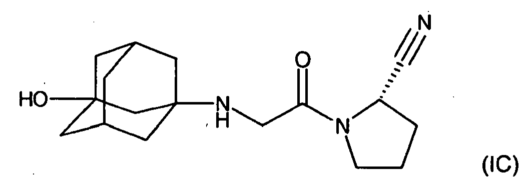 also referred to as pyrrolidine, 1-[3-hydroxy-1-adamantyl)amino] acetyl-2-cyano-, (S) and its pharmaceutically acceptable acid addition salts.

Unless otherwise specified herein, common definitions are intended by the words and terms used herein. As throughout this specification the singular is intended to include the plural and vice versa.

The term "therapeutically effective amount" shall mean that amount of compound that will elicit the biological or medical response of a tissue, system or animal (mammal) that is being sought by a researcher or clinician.

The terms "mammal", "mammalian organism", "subject" or "patient" are used interchangeably herein and include, but are not limited to, humans, dogs, cats, horses, pigs, cows, monkeys, rabbits, mice and laboratory animals. The preferred mammals are humans. The term "modulate" refers to the treating, prevention, suppression, enhancement or induction of a function or condition. For example, the compounds of the present invention can modulate hyperlipidemia by lowering cholesterol in a human, thereby suppressing hyperlipidemia.

The term "treating" means the management and care of a human subject for the purpose of combating the disease, condition or disorder and includes the administration of a compound of the present invention to prevent the onset of the symptoms or complications, alleviating the symptoms or complications, or eliminating the disease, condition or disorder.

The term "elevated levels of Lp(a)" as used herein shall mean levels of Lp(a) which subjects the patient to the risk of vascular, particularly cardiovascular diseases, mediated by Lp(a), including but not limited to CHD, ischemic stroke, restenosis after angioplasty, peripheral vascular disease, intermittent claudication, myocardial infarction (e.g. reduction in necrosis), dyslipidemia and post-prandial lipemia.

The term "hyperlipidemia" refers to the presence of an abnormally elevated level of lipids in the blood. Hyperlipidemia can appear in at least three forms: (1) hypercholesterolemia, i.e., an elevated cholesterol level; (2) hypertriglyceridemia, i.e., an elevated triglyceride level; and (3) combined hyperlipidemia, i.e., a combination of hypercholesterolemia and hypertriglyceridemia. This term also refers to elevated levels of one or more lipoproteins, e.g., elevated levels of Lp(a), LDL and/or VLDL.

The term "cholesterol" refers to a steroid alcohol that is an essential component of cell membranes and myelin sheaths and, as used herein, incorporates its common usage. Cholesterol also serves as a precursor for steroid hormones and bile acids.

The term "triglyceride(s)" (TGs), as used herein, incorporates its common usage. TGs consist of three fatty acid molecules esterified to a glycerol molecule and serve to store fatty acids which are used by muscle cells for energy production or are taken up and stored in adipose tissue.

Because cholesterol and TGs are water insoluble, they must be packaged in special molecular complexes known as "lipoproteins" in order to be transported in the plasma. Lipoproteins can accumulate in the plasma due to overproduction and/or deficient removal. There are at least five distinct lipoproteins differing in size, composition, density and function. In the cells of the small of the intestine, dietary lipids are packaged into large lipoprotein complexes called "chylomicrons", which have a high TG and low cholesterol content. In the liver, TG and cholesterol esters are packaged and released into plasma as TG-rich lipoprotein called VLDL, whose primary function is the endogenous transport of TGs made in the liver or released by adipose tissue. Through enzymatic action, VLDL can be either reduced and taken up by the liver, or transformed into IDL. IDL, is in turn, either taken up by the liver, or is further modified to form the LDL. LDL is either taken up and broken down by the liver, or is taken up by extrahepatic tissue. HDL helps remove cholesterol from peripheral tissues in a process called reverse cholesterol transport.

Exemplary primary hyperlipidemia include, but are not limited to, the following:
1) Familial hyperchylomicronemia, a rare genetic disorder which causes a deficiency in an enzyme, LP lipase, that breaks down fat molecules. The LP lipase deficiency can cause the accumulation of large quantities of fat or lipoproteins in the blood;
2) Familial hypercholesterolemia, a relatively common genetic disorder caused where the underlying defect is a series of mutations in the LDL receptor gene that result in malfunctioning LDL receptors and/or absence of the LDL receptors. This brings about ineffective clearance of LDL by the LDL receptors resulting in elevated LDL and total cholesterol levels in the plasma;
3) Familial combined hyperlipidemia, also known as multiple lipoprotein-type hyperlipidemia; an inherited disorder where patients and their affected first-degree relatives can at various times manifest high cholesterol and high triglycerides. Levels of HDL cholesterol are often moderately decreased;
4) Familial defective apolipoprotein B-100 is a relatively common autosomal dominant genetic abnormality. The defect is caused by a single nucleotide mutation that produces a substitution of glutamine for arginine which can cause reduced affinity of LDL particles for the LDL receptor. Consequently, this can cause high plasma LDL and total cholesterol levels;
5) Familial dysbetaliproteinemia, also referred to as Type III hyperlipoproteinemia, is an uncommon inherited disorder resulting in moderate to severe elevations of serum TG and cholesterol levels with abnormal apolipoprotein E function. HDL levels are usually normal; and
6) Familial hypertriglyceridemia, is a common inherited disorder in which the concentration of plasma VLDL is elevated. This can cause mild to moderately elevated triglyceride levels (and usually not cholesterol levels) and can often be associated with low plasma HDL levels. Risk factors in exemplary secondary hyperlipidemia include, but are not limited to, the following: (1) disease risk factors, such as a history of Type 1 diabetes, Type 2 diabetes, Cushing's syndrome, hypothyroidism, cholestasis and certain types of renal failure; (2) drug risk factors, which include, birth control pills; hormones, such as estrogen and corticosteroids; certain diuretics; and various β-blockers; (3) dietary risk factors include dietary fat intake per total calories greater than 40%; saturated fat intake per total calories greater than 10%; cholesterol intake greater than 300 mg per day; habitual and excessive alcohol use; bulimia, anorexia nervosa, and obesity.

"Pharmaceutically acceptable salt(s)" refer to the non-toxic alkali metal, alkaline earth metal, and ammonium salts commonly used in the pharmaceutical industry including the sodium, potassium, lithium, calcium, magnesium, barium, ammonium and protamine zinc salts, which are prepared by methods well-known in the art. The term also includes non-toxic acid addition salts, which are generally prepared by reacting the compounds of the present invention with a suitable organic or inorganic acid. Representative salts include, but are not limited to, the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, napsylate and the like.

"Pharmaceutically acceptable acid addition salt(s)" refers to those salts which retain the biological effectiveness and properties of the free bases and which are not biologically or otherwise undesirable, formed with inorganic acids, such as hydrochloric acid, hydrobromic acid, sulfuric acid, nitric acid, phosphoric acid and the like; and organic acids, such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, salicylic acid and the like. For a description of pharmaceutically acceptable acid addition salts as prodrugs see, e.g., Bundgaard, Ed., Design of Prodrugs, Elsevier Science Publishers, Amsterdam (1985)).

Especially preferred is the compound of formula IC: 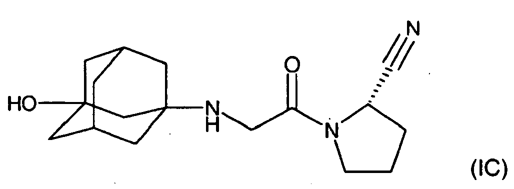 and its pharmaceutically acceptable acid addition salts.

Included with the scope of the present invention are pharmaceutically acceptable salts and pharmaceutically acceptable acid addition salts of the compounds of formula IC. Compound IC, is disclosed in U.S. Patent No. 6, 166,063 issued December 26, 2000 and PCT publication WO 00/34241 published June 15, 2000.

Conditions associated with hyperlipidemia include, atherosclerosis, angina pectoris, carotid artery disease, cerebral arteriosclerosis, xanthoma, CHD, ischemic stroke, restenosis after angioplasty, peripheral vascular disease, intermittent claudication, myocardial infarction, dyslipidemia, post-prandial lipemia. The compound of formula I is compound of formula IC.

The compounds of formula I, and their corresponding pharmaceutically acceptable acid addition salts, may be combined with one or more pharmaceutically acceptable carriers and, optionally, one or more other conventional pharmaceutical adjuvants and administered enterally, e.g., orally, in the form of tablets, capsules, caplets, etc. or parenterally, e.g., intravenously, in the form of sterile injectable solutions or suspensions. The enteral and parenteral compositions may be prepared by conventional means.

The compounds of formula I, and their corresponding pharmaceutically acceptable acid addition salts, may be formulated into enteral and parenteral pharmaceutical compositions containing an amount of the active substance that is effective for modulating, treating or preventing hyperlipidemia and conditions associated with hyperlipidemia and for lowering levels of Lp(a), LDL and/or VLDL, in unit dosage form and such compositions comprising a pharmaceutically acceptable carrier.

The compounds of formula I, including those of each of the subscopes thereof and each of the examples, may be administered in enantiomerically pure form, e.g. >98%, preferably >99%; or together with the R enantiomer, e.g., in racemic form. The above dosage ranges are based on the compounds of formula I (excluding the amount of the R enantiomer).

The precise dosage of the compounds of formula I, and their corresponding pharmaceutically acceptable acid addition salts, to be employed for modulating, treating or preventing hyperlipidemia and conditions associated with hyperlipidemia, and for lowering levels of Lp(a), LDL and/or VLDL, depends upon several factors, including the host, the nature and the severity of the condition being treated, the mode of administration and the particular compound employed. However, in general, hyperlipidemia and conditions associated with hyperlipidemia are effectively treated when compounds of formula I, or a corresponding pharmaceutically acceptable acid addition salt, is administered enterally, e.g., orally or parenterally, e.g., intravenously, preferably orally, at a daily dosage of 0.002-5 mg/kg, preferably 0.02-2.5 mg/kg body weight or, for most larger primates, a daily dosage of 0.1-250 mg/kg, preferably 1-100 mg/kg. A typical oral dosage unit is 0.01-0.75 mg/kg, one to three times a day. Usually, a small dose is administered initially and the dosage is gradually increased until the optimal dosage for the host under treatment is determined. The upper limit of dosage is that imposed by side effects and can be determined by trial for the host being treated.

The compounds of the present invention can be used effectively alone or in combination with one or more additional active agents depending on the desired target therapy. The use of combination preparations comprising compound IC are not within the claimed scope of the present invention. A number of studies have investigated the benefits of combination therapies with oral agents (see, e.g., Mahler, J. Clin. Endocrinol. Metab., Vol. 84, pp. 1165-1171 (1999); United Kingdom Prospective Diabetes Study Group: UKPDS 28, Diabetes Care, Vol. 21, pp. 87-92 (1998); Bardin, Ed., Current Therapy in Endocrinology and Metabolism, 6th Edition, Mosby-Year Book, Inc., St. Louis, MO (1997); Chiasson et al., Ann. Intern. Med., Vol. 121, pp. 928-935 (1994); Coniff et al., Clin. Ther., Vol. 19, pp. 16-26 (1997); Coniff et al., Am. J. Med., Vol. 98, pp. 443-451 (1995); Iwamoto et al, Diabet. Med., Vol. 13, pp. 365-370 (1996); and Kwiterovich, Am. J. Cardiol., Vol. 82, No. 12A, pp. 3U-17U (1998)). These studies indicate that diabetes and hyperlipidemia modulation can be further improved by the addition of a second agent to the therapeutic regimen. Combination therapy includes administration of a single pharmaceutical dosage formulation which contains a compound of formula I (formula IC) and one or more additional active agents, as well as administration of a compound of formula I ( formula IC) and each active agent in its own separate pharmaceutical dosage formulation. For example, a compound of formula I and an HMG-CoA reductase inhibitor can be administered to the human subject together in a single oral dosage composition, such as a tablet or capsule, or each agent can be administered in separate oral dosage formulations. Where separate dosage formulations are used, a compound of formula I and one or more additional active agents can be administered at essentially the same time, i.e., concurrently; or at separately staggered times, i.e., sequentially. Combination therapy is understood to include all these regimens.

A pharmaceutical composition as described above comprising a quantity which is jointly therapeutically effective against herein mentioned diseases of a combination as described above and at least one pharmaceutically acceptable carrier.

The structure of the active agents identified by generic or tradenames may be taken from the actual edition of the standard compendium "The Merck Index" or from databases, e.g. Patents International (e.g. IMS World Publications). Any person skilled in the art is fully enabled to identify the active agents and, based on these references, likewise enabled to manufacture and test the pharmaceutical indications and properties in standard test models, both in vitro and in vivo.

The corresponding active ingredients or a pharmaceutically acceptable salts thereof may also be used in form of a solvate, such as a hydrate or including other solvents, used for crystallization.

The compounds to be combined can be present as pharmaceutically acceptable salts. If these compounds have, for example, at least one basic center, they can form acid addition salts. Corresponding acid addition salts can also be formed having, if desired, an additionally present basic center. The compounds having an acid group (for example COOH) can also form salts with bases.

All the more surprising is the experimental finding that the combined administration of formula I or a salt thereof and a therapeutic agent (active agent) selected from the group mentioned below results not only in a beneficial, especially a synergistic, therapeutic effect, but also in additional benefits resulting from the combined treatment and further surprising beneficial effects compared to a monotherapy applying only one of the pharmaceutically active compounds used in the combinations disclosed herein.

It can be shown by established test models and especially those test models described herein that the combination of the compound of formula (I) with a therapeutic agent selected from the group described herein results in a more effective prevention or preferably treatment of diseases specified herein. In particular, it can be shown by established test models and especially those test models described herein that combination results in a more effective prevention or preferably treatment of diseases specified hereinafter.

If taken simultaneously, this results not only in a further enhanced beneficial, especially a synergistic, therapeutic effect, but also in additional benefits resulting from the simultaneous treatment such as a surprising prolongation of efficacy, a broader variety of therapeutic treatment and surprising beneficial effects, e.g. less increase of weight, on diseases and conditions associated with diabetes mellitus, for a number of combinations as described herein. Moreover, for a human patient, especially for elderly people, it is more convenient and easier to remember to take two tablets at the same time, e.g. before a meal, than staggered in time, i.e. according to a more complicated treatment schedule. More preferably, both active ingredients are administered as a fixed combination, i.e. as a single tablet, in all cases described herein. Taking a single tablet is even easier to handle than taking two tablets at the same time. Furthermore, the packaging can be accomplished with less effort.

The person skilled in the pertinent art is fully enabled to select a relevant and standard animal test model to prove the hereinbefore and hereinafter indicated therapeutic indications and beneficial effects.

The pharmaceutical activities as effected by administration of the compounds of formula (I) or of the combination of the active agents can be demonstrated e.g. by using corresponding pharmacological models known in the pertinent art.

Further benefits when applying the composition of the present invention are that lower doses of the individual drugs to be combined can be used to reduce the dosage, for example, that the dosages need not only often be smaller but are also applied less frequently, or can be used in order to diminish the incidence of side effects. This is in accordance with the desires and requirements of the patients to be treated.

Preferably, the jointly therapeutically effective amounts of the active agents can be administered simultaneously or sequentially in any order, separately or in a fixed combination.

A further example of a preferred combination therapy can be seen in modulating hyperlipidemia, wherein the compounds of formula I can be effectively used in combination with, for example, statins, i.e., fluvastatin, lovastatin, pravastatin, atorvastatin or simvastatin; bile acid-binding resins, i.e., colestipol or cholestyramine; nicotinic acid, probucol, β-carotene, vitamin E or vitamin C. The compound of formula I is compound of formula IC. Preferably the active agent (b) is selected from the group consisting of fluvastatin,lovastatin, pravastatin, atorvastatin or simvastatin.

These pharmaceutical preparations are for enteral, such as oral, and also rectal or parenteral, administration to homeotherms, with the preparations comprising the pharmacological active compound either alone or together with customary pharmaceutical auxiliary substances. For example, the pharmaceutical preparations consist of from about 0.1 % to 90 %, preferably of from about 1 % to about 80 %, of the active compound. Pharmaceutical preparations for enteral or parenteral, and also for ocular, administration are, for example, in unit dose forms, such as coated tablets, tablets, capsules or suppositories and also ampoules. These are prepared in a manner that is known per se, for example using conventional mixing, granulation, coating, solubulizing or lyophilizing processes. Thus, pharmaceutical preparations for oral use can be obtained by combining the active compound with solid excipients, if desired granulating a mixture which has been obtained, and, if required or necessary, processing the mixture or granulate into tablets or coated tablet cores after having added suitable auxiliary substances.

The dosage of the active compound can depend on a variety of factors, such as mode of administration, homeothermic species, age and/or individual condition.

Preferred dosages for the active ingredients of the pharmaceutical combination are therapeutically effective dosages, especially those which are commercially available.

Normally, in the case of oral administration, an approximate daily dose of from about 1 mg to about 360 mg is to be estimated e.g. for a patient of approximately 75 kg in weight.

The dosage of the active compound can depend on a variety of factors, such as mode of administration, homeothermic species, age and/or individual condition.

The pharmaceutical preparation will be supplied in the form of suitable dosage unit form, for example, a capsule or tablet, and comprising an amount, being together with the further component(s) jointly effective, e.g.

The doses of compounds of formula (I) to be administered to warm-blooded animals, for example human beings, of, for example, approximately 70 kg body weight, especially the doses effective in the inhibition of the enzyme renin, e.g. in lowering blood pressure and/or in improving the symptoms of glaucoma, are from approximately 3 mg to approximately 3g, preferably from approximately 10mg to approximately 1 g, for example approximately from 20mg to 200mg, per person per day, divided preferably into 1 to 4 single doses which may, for example, be of the same size. Usually, children receive about half of the adult dose. The dose necessary for each individual can be monitored, for example by measuring the serum concentration of the active ingredient, and adjusted to an optimum level. Single doses comprise, for example, 10, 40 or 100 mg per adult patient.

### EXAMPLES

The present invention is further described by the following example.

### 1. Example 1

### Evaluation of the effects of compound IC on human lipid profiles

Sixty (60) patients comprised of male and non-fertile female patients aged at least 30 years with a diagnosis of Type 2 diabetes mellitus of at least three months duration, who have been treated with diet alone for at least one month prior to study entry were selected. The study was broken down into two periods. Period 1 was the four weeks prior to the beginning of the study, with period 2 being four weeks and being the actual study period when patients were treated with compound IC. Accordingly, study entry was Week -4 and the endpoint was after the fourth week of Period 2.

Patients were randomized in a ratio of 1:1:1 as follows: compound IC at 200 mg once a day (OD), compound IC at 100 mg OD and placebo. The patients received compound IC 30 minutes before breakfast. There were 5 test days in the study. Patients attended as outpatients for fasting blood sampling at Week -4 (study entry), Week -2 and Week 2 and as inpatients for 24 hours on Week 0 (= baseline) and Week 4 (= endpoint). On the two inpatient test days, the total caloric intake of breakfast, lunch and dinner was standardized and standard test meals were administered in place of breakfast and dinner. Triglycerides, total cholesterol and lipid fractions (LDL, VLDL and HDL) were measured during 24 hours following the breakfast standard meal.

On the three outpatient test days, patients fasted for at least 7 hours (i.e., no food or drinks (except water) after midnight on the day before the scheduled visit) and attended between 07.00 and 10.00 h and did not take the morning dose of compound IC.

On the two inpatient test days, patients fasted for at least 7 hours, i.e., no food or drinks (except water) after midnight on the day before the scheduled visit, and attended the clinic at 07.00 h. On each of the two test days, the total caloric intake during 24 hours was standardized and standard test meals were administered for breakfast (about 08.00 h) and dinner (about 18.00 h). Lunch was taken at approximately 13.00 h. On Day 1, no compound IC was administered but at Week 4, patients took compound IC as normal, 30 minutes before the standard breakfast. Triglycerides, total cholesterol and lipid fractions (LDL, VLDL and HDL) were evaluated. Triglycerides, total cholesterol and HDL were measured and LDL and VLDL calculated according to the method of Friedewald et al., "Estimation of the Concentration of Low-Density Lipoprotein Cholesterol Without the Use of the Preparative Centrifuge", Clin. Chem., Vol. 18, No. 6, pp. 499-502 (1972).

Illustrative of the invention, compound IC markedly lowered levels of triglyceride, total cholesterol, LDL and VLDL compared to placebo.

## Claims

1. Use of an active ingredient consisting of a compound of formula IC 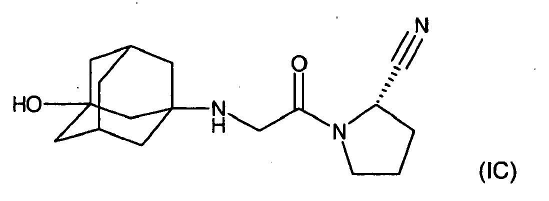 in free form or in form of a pharmaceutically acceptable acid addition salt,
for the manufacture of a medicament for modulating hyperlipidemia.

2. Use according to claim 1, wherein 20 mg to 200 mg of a compound of formula IO has been administered daily.

3. Use according to claim 1 or claim 2, for the manufacture of a medicament for modulating hypercholesterolemia.

4. Use according to claim 1 or claim 2, for the manufacture of a medicament for modulating hypertriglyceridemia,

5. Use according to claim 1 or claim 2, for the manufacture of a medicament for modulating combined hyperlipidemia.

6. Use according to claim 1 or claim 2, for the manufacture of a medicament for modulating hyperchylomicronemia.

7. Use according to claim 1 or claim 2, for the manufacture of a medicament for modulating defective apolipoprotein B-100.

8. Use according to claim 1 or claim 2, for the manufacture of a medicament for modulating defective dysbetaliproteinemia B-100.

## Patentansprüche

1. Verwendung eines Wirkstoffs, der besteht aus einer Verbindung der Formel IC 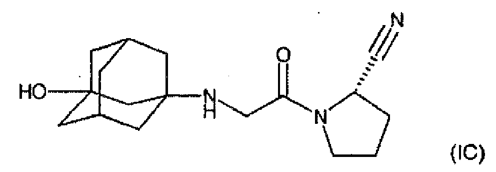 in freier Form oder in Form eines pharmazeutisch akzeptablen Säureadditionssalzes, zur Herstellung eines Arzneimittels für eine Modulation von Hyperlipidämie.

2. Verwendung nach Anspruch 1, worin 20 mg bis 200 mg einer Verbindung der Formel IC täglich verabreicht werden.

3. Verwendung nach Anspruch 1 oder 2 zur Herstellung eines Arzneimittels für eine Modulation von Hypercholesterolämie.

4. Verwendung nach Anspruch 1 oder 2 zur Herstellung eines Arzneimittels für eine Modulation von Hypertriglyceridämie.

5. Verwendung nach Anspruch 1 oder 2, zur Herstellung eines Arzneimittels für eine Modulation von kombinierter Hyperlipidämie.

6. Verwendung nach Anspruch 1 oder 2 zur Herstellung eines Arzneimittels für eine Modulation von Hyperchylomikronämie.

7. Verwendung nach Anspruch 1 oder 2 zur Herstellung eines Arzneimittels für eine Modulation von defektivem Apolipoprotein B-100.

8. Verwendung nach Anspruch 1 oder 2 zur Herstellung eines Arzneimittels für eine Modulation von defektiver Dysbetalipoproteinämie B-100.

## Revendications

1. Utilisation d'un principe actif constitué par un composé de formule IC 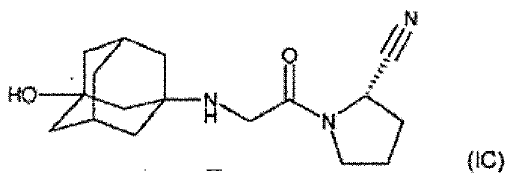 sous forme libre ou sous la forme d'un sel d'addition d'acide pharmaceutiquement acceptable, pour la fabrication d'un médicament destiné à moduler l'hyperlipidémie.

2. Utilisation selon la revendication 1, dans laquelle de 20 mg à 200 mg d'un composé de formule IC ont été administrés journalièrement.

3. Utilisation selon la revendication 1 ou la revendication 2, pour la fabrication d'un médicament destiné à moduler l'hypercholestérolémie.

4. Utilisation selon la revendication 1 ou la revendication 2, pour la fabrication d'un médicament destiné à moduler l'hypertriglycéridémie.

5. Utilisation selon la revendication 1 ou la revendication 2, pour la fabrication d'un médicament destiné à moduler l'hyperlipidémie combinée.

6. Utilisation selon la revendication 1 ou la revendication 2, pour la fabrication d'un médicament destiné à moduler l'hyperchylomicronémie.

7. Utilisation selon la revendication 1 ou la revendication 2, pour la fabrication d'un médicament destiné à moduler la déficience en apolipoprotéine B-100.

8. Utilisation selon la revendication 1 ou la revendication 2, pour la fabrication d'un médicament destiné à moduler la dysbêtalipoprotéinémie.
